# EUROPÄISCHE PATENTSCHRIFT

(11) **EP 1 304 095 B1**
(45) Veröffentlichungstag und Bekanntmachung des Hinweises auf die Patenterteilung: **30.03.2011**
(21) Anmeldenummer: 02022405.1
(22) Anmeldetag: 04.10.2002
(51) Int. Cl.: A61F 13/20

(54) **Verfahren sowie Vorrichtung zum Herstellen eines Tampons**
Method and apparatus for making a tampon
Procédé et appareil de fabrication d'un tampon

(30) Priorität: 19.10.2001 DE 10151758
(43) Veröffentlichungstag der Anmeldung: 23.04.2003
(73) Patentinhaber: Ruggli Projects AG, 6332 Hagendorn Cham (CH)
(72) Erfinder: Rolli, Kilian, 5408 Ennetbaden (CH); Kälin, Christoph, 6314 Unterägeri (CH)
(74) Vertreter: Ofner, Clemens

(56) Entgegenhaltungen:
- WO-A-00/53141
- US-A- 2 263 909
- US-A- 4 109 354
- US-A- 4 498 218

## Beschreibung

Die Erfindung betrifft ein Verfahren zum Herstellen eines Tampons, bei dem in einer Tamponpresse mit radial beweglichen Pressbacken streifenförmige Abschnitte eines zylindrischen Rohlings aus saugfähigem Material bis zur Bildung eines Vorformlings gepresst werden, der sich, im Querschnitt gesehen, aus einem zentralen Tamponkern und sich von dem Tamponkern radial nach aussen erstreckenden Längsrippen zusammensetzt, die durch Längsrillen voneinander getrennt sind.

Die Erfindung betrifft ferner eine Vorrichtung zum Herstellen eines Tampons, mit einer Tamponpresse aus über den Umfang eines zu pressenden Tamponrohlings verteilt angeordneten, vor- und zurückbeweglichen und mit hervorstehenden Pressschneiden versehenen Pressbacken.

Tampons bestehen üblicherweise aus einem Faservlies, das zunächst zu einem Tamponrohling gewickelt und anschliessend auf die gewünschte Endform des Tampons gepresst wird. Anforderungen an einen Tampon sind vornehmlich eine hohe Formsteifigkeit und Saugleistung. Während die Formsteifigkeit von der durch die Höhe der Verdichtung des Faservlies beim Pressen erzeugten Knickfestigkeit abhängt, wird die Saugleistung durch die von dem Tampon bewirkte Sauggeschwindigkeit und Absorptionsfähigkeit bestimmt. Letztere lassen sich dann in hohem Masse erreichen, wenn das Faserflies eine hohe Kapillarität aufweist und demzufolge von einer gering verdichteten, weichen Faserstruktur ist.

Zur Erzeugung derartiger Tampons ist aus der EP 0 639 363 A2 eine Tamponpresse bekannt, bei der die Presswerkzeuge jeweils am innenliegenden Ende eines Presshebels angeordnet sind, der zwischen zwei Endstellungen verschwenkbar ist. Die gleichmässig über den Umfang verteilten Presshebel sind an ihren aussenliegenden Enden jeweils über einen Koppelhebel an einem Ring gelagert, wohingegen eine mittige gelenkige Lagerung der einzelnen Presshebel an einem Verstellring erfolgt, der zugleich den Antrieb der Tamponpresse übernimmt. Mit dieser bekannten Tamponpresse lassen sich zwar qualitativ gute Tampons herstellen, jedoch ist die Antriebsleistung für den Antrieb der radial vor- und zurückbeweglichen Pressbacken relativ hoch, was auf Dauer ferner zu einem hohen Verschleiss der beweglichen Teile der Tamponpresse führen kann.

Der Erfindung liegt daher die Aufgabe zugrunde, ein Verfahren sowie eine Vorrichtung zum Pressen eines Tampons dahingehend weiterzubilden, dass auch ohne eine im Vergleich zum Stand der Technik erhöhte Antriebsleistung eine höhere Presskraft auf den Tamponrohling erzielt wird.

Diese Aufgabe wird bei einem Verfahren mit den eingangs genannten Merkmalen dadurch gelöst, dass die Kraft zur Bewegung jeder Pressbacke über ein zu der Pressbacke gelenkiges Druckübertragungselement auf die jeweilige Pressbacke übertragen wird, und dass die Richtung der Krafteinleitung zu einem Zeitpunkt des Pressvorgangs gleichgerichtet zu der Bewegung der Pressbacke ist.

Es hat sich herausgestellt, dass durch ein Tampon-Pressverfahren mit diesen Merkmalen hohe Presskräfte bei zugleich relativ geringer Antriebsleistung auf den Tamponrohling ausgeübt werden können. Der Kraftverlauf innerhalb der verwendeten Tamponpresse ist in sich geschlossen, zum Schliessen der Tamponpresse reicht bereits eine relativ geringe Antriebsleistung aus.

Zur Lösung der Aufgabenstellung wird ferner eine Vorrichtung vorgeschlagen, die ausser durch die eingangs genannten Vorrichtungsmerkmale dadurch gekennzeichnet ist, dass die

Pressbacken in gelenkiger Verbindung mit dem einen Ende eines Druckübertragungselementes stehen, dessen anderes Ende mit einem Antrieb in Verbindung steht und dass in einer Vorschubposition der jeweiligen Pressbacke die Kraftübertragungsrichtung des Druckübertragungselements gleichgerichtet zu der Bewegung der Pressbacke ist.

Ausgestaltungen des erfindungsgemässen Verfahrens sowie der erfindungsgemässen Vorrichtung sind in den Unteransprüchen angegeben.

Vorzugsweise wird das erfindungsgemässe Verfahren mit einer Tamponpresse durchgeführt, bei der die einzelnen Pressbacken jeweils am Ende eines gelenkig gelagerten Presshebels angeordnet sind. Hierzu wird mit einer bevorzugten Ausgestaltung vorgeschlagen, dass die streifenförmigen Abschnitte durch Verschwenken von mit den Pressbacken versehenen, gelenkig mit dem Druckübertragungselement verbundenen Presshebeln um Gelenke gepresst werden, wobei die Lage aller Gelenke unveränderlich zueinander ist, und dass zu dem genannten Zeitpunkt des Pressvorgangs die Richtung der Krafteinleitung quer zu der gedachten Verbindungslinie zwischen Gelenk und Pressbacke ist. Vorzugsweise wird die Kraft zum Verschwenken des Presshebels an einem Ort des Presshebels zwischen seinem Gelenk und seiner Pressbacke in den Presshebel eingeleitet.

Mit einer bevorzugten Ausgestaltung des erfindungsgemässen Verfahrens wird z.B. vorgeschlagen, dass bei maximalem Vorschub der Pressbacken die Krafteinleitung radial auf den Vorformling ausgerichtet ist. Bei vorgegebener Antriebsleistung der Tamponpresse wird auf diese Weise die höchste Presskraft in jenem Augenblick erreicht, in dem auch die Verdichtung des Tamponrohlings und damit verbunden die Verformkräfte am höchsten sind.

In weiterer Ausgestaltung des erfindungsgemässen Verfahrens wird vorgeschlagen, dass die Lagerachsen für die gelenkige Lagerung der Presshebel feststehend sind. Hierdurch wird bewirkt, dass die Presshebel während des Pressvorganges eine reine Schwenkbewegung ausführen, hingegen keine Lateralbewegung des Presshebels insgesamt eintritt. Auf diese Weise lassen sich die Presskräfte auf den Tamponrohling besser kontrollieren, als dies z. B. bei der Tamponpresse gemäss der EP 0 639 363 A2 der Fall ist.

Vorzugsweise erfolgt die Betätigung aller Preßbacken durch Verdrehen eines gemeinsamen Antriebselements um eine Drehachse, die mit der Mittelachse des Vorformlings zusammenfällt.

Gemäß einer bevorzugten Ausgestaltung der erfindungsgemäßen Vorrichtung ist der Ort des den Preßhebel mit dem Druckübertragungselement verbindenden Gelenks in Längsrichtung des Preßhebels einstellbar. Dadurch wird die Möglichkeit eröffnet, durch entsprechende Änderungen an der Geometrie der einzelnen Bauteile unterschiedliche Materialqualitäten und etwaige Gewichtsunterschiede der verarbeiteten Tamponrohlinge zu berücksichtigen, indem die erzielten Preßkräfte entsprechend angepaßt werden. Es läßt sich daher ein- und dieselbe Tamponpresse durch geringe Änderungen für die Verarbeitung unterschiedlicher Tampons einsetzen, was im Ergebnis zu sehr universellen Einsatzmöglichkeiten der erfindungsgemäßen Vorrichtung führt.

Einzelheiten und weitere Vorteile des Gegenstandes der Erfindung ergeben sich aus der nachfolgenden Beschreibung eines bevorzugten Ausführungsbeispieles. In den Zeichnungen zeigen im Einzelnen:
- Figur 1: eine schematische Ansicht einer Vorrichtung zum Pressen eines Tampons mit Preßwerkzeugen in einer völlig geöffneten Stellung;
- Figur 2: eine schematische Ansicht der Vorrichtung nach Figur 1 in einer geringfügig geschlossenen Haltestellung;
- Figur 3: eine schematische Ansicht der Vorrichtung nach Figur 1 in maximal geschlossener Preßstellung;
- Figur 4: eine schematische Ansicht der Vorrichtung nach Figur 1 in einer dem Preßvorgang folgenden, leicht gelüfteten Stellung und
- Figur 5: in einer gegenüber den Figuren 1 bis 4 vergrößerten Teildarstellung Einzelheiten des bei der Vorrichtung verwendeten Druckstabes sowie Variationsmöglichkeiten hinsichtlich des Gelenks zwischen Druckstab und zugehörigem Preßhebel.

Die auf der Zeichnung dargestellte Vorrichtung zum Pressen eines Tampons weist insgesamt acht gleich ausgebildete Preßwerkzeuge auf, die gleichmäßig verteilt über den Umfang eines zu pressenden Tamponrohlings 1 angeordnet sind. Diese Anzahl kann auch von acht abweichen. Bestandteile der Preßwerkzeuge sind Preßbacken 2 mit daran zusätzlich angeformten Preßschneiden 3. Die Preßbacken 2 mit den Preßschneiden 3 lassen sich in einer im wesentlichen radialen Schwenkbewegung auf den Tamponrohling 1 zu bewegen, um diesen durch in erster Linie radiale Kräfte zu pressen und hierbei einen sog. Vorformling zu erzeugen. Die an den Preßbacken 2 ausgebildeten Preßschneiden 3 führen hierbei zur Bildung von Längsrillen in dem Tampon. Nach dem Abschluß des Preßverfahrens setzt sich der Vorformling aus einem zentralen Tamponkern und sich von dem Tamponkern radial nach außen erstreckenden Längsrippen zusammen, wobei die Längsrippen durch die durch die Preßschneiden 3 erzeugten Längsrillen voneinander getrennt sind.

Jede der Preßbacken 2 ist an dem freien Ende eines Preßhebels 4 befestigt und ist im wesentlichen rechtwinklig zu dem Preßhebel 4 ausgerichtet. Diese Befestigung kann einstückig sein, alternativ lassen sich die einzelnen Preßbacken 2 auch auswechselbar am Ende der Preßhebel 4 befestigen, um so die Preßbacken gegen andere Preßbacken austauschen zu können.

Die vorzugsweise gekrümmt gestalteten Preßhebel 4 sind an ihrem anderen Ende auf einer ein Gelenk bildenden Lagerachse 5 gelagert. Die Lagerachsen 5 sämtlicher Preßhebel 4 befinden sich auf einem feststehenden Stützring 6 oder einem anderen feststehenden Bauteil der Tamponpresse. Hierbei sind alle Lagerachsen 5 auf einem gemeinsamen, gedachten Kreis 7 fixiert, so daß die Lage aller Lagerachsen 5 der Preßhebel zueinander unveränderlich ist.

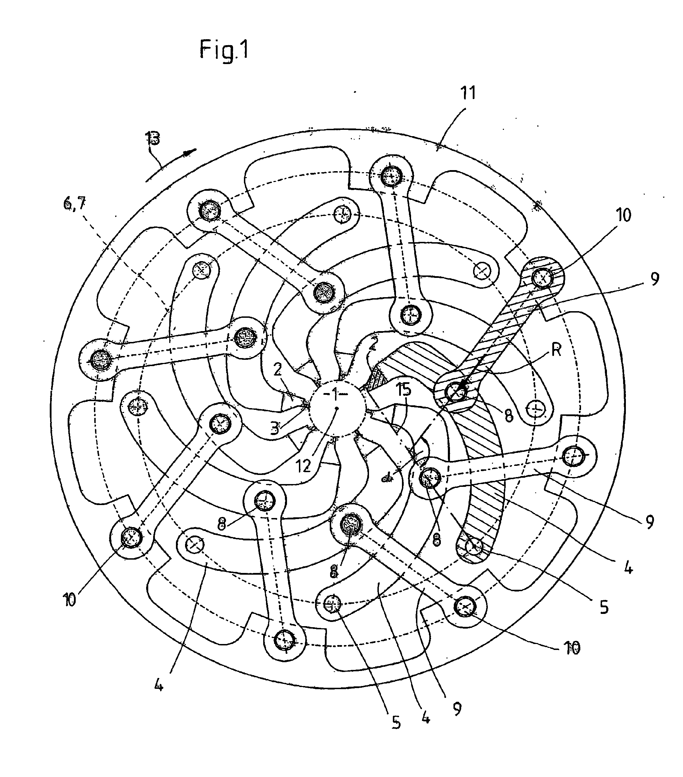

Zur Verdeutlichung ist in Figur 1 einer der insgesamt acht Preßhebel 4 durch eine entsprechende Schraffierung hervorgehoben.

Die Preßhebel 4 sind mit daran befestigten Drehzapfen 8 versehen, über die die Preßhebel gelenkig mit Druckstäben 9 verbunden sind, die als Druckübertragungselemente dienen. Vorzugsweise befindet sich auf jeder Seite der Preßhebel 4 jeweils ein Druckstab 9, um so eine querkraftfreie Krafteinleitung von den Druckstäben 9 auf den Preßhebel zu ermöglichen. Der Drehzapfen 8 bildet ein Gelenk, welches, wie Figur 1 erkennen läßt, näher an der Preßbacke 2 denn an der Lagerachse 5 des Preßhebels 4 sitzt. Beim Ausführungsbeispiel befindet sich das durch den Drehzapfen 8 gebildete Gelenk in etwa im zweiten Drittel der Länge des gebogenen Preßhebels 4, von dessen Lagerachse 5 aus betrachtet.

Auch das andere Ende des Druckstabes 9 ist gelenkig gelagert und zwar an einem Drehzapfen 10 eines ringförmigen Antriebselementes 11. An dem ringförmigen Antriebselement 11 sind in entsprechender Weise, gleichmäßig über den Umfang verteilt, auch die Drehzapfen 10 für alle übrigen Druckstäbe 9 angeordnet. Das ringförmige Antriebselement 11 ist um eine Drehachse 12 gelagert, die mit der Mittelachse des Tampons 1 zusammenfällt, so daß die beschriebene Tamponpresse insgesamt völlig symmetrisch aufgebaut ist. Insbesondere bewegt sich das ringförmige Antriebselement 11 konzentrisch nicht nur zu dem Tampon 1, sondern auch zu dem Kreis 7, auf dem die feststehenden Lagerachsen 5 für die Preßhebel 4 angeordnet sind.

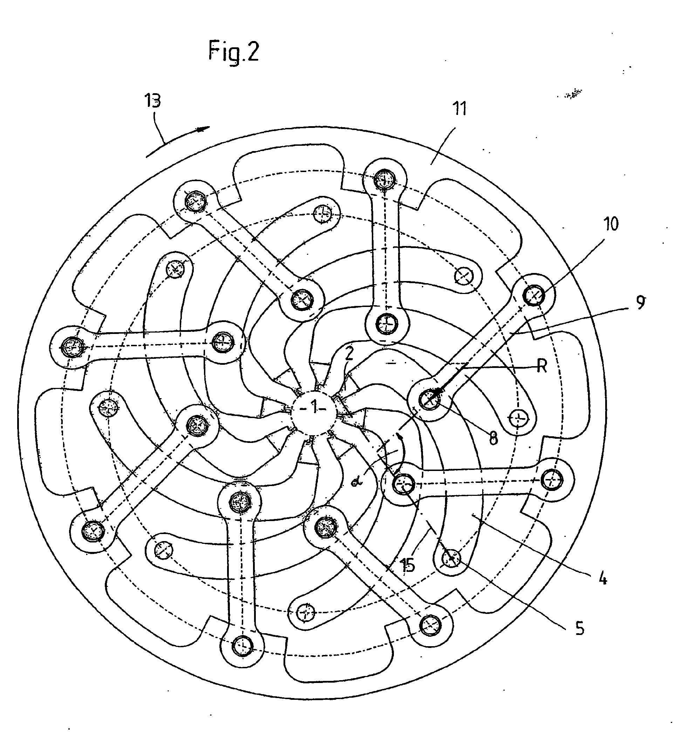

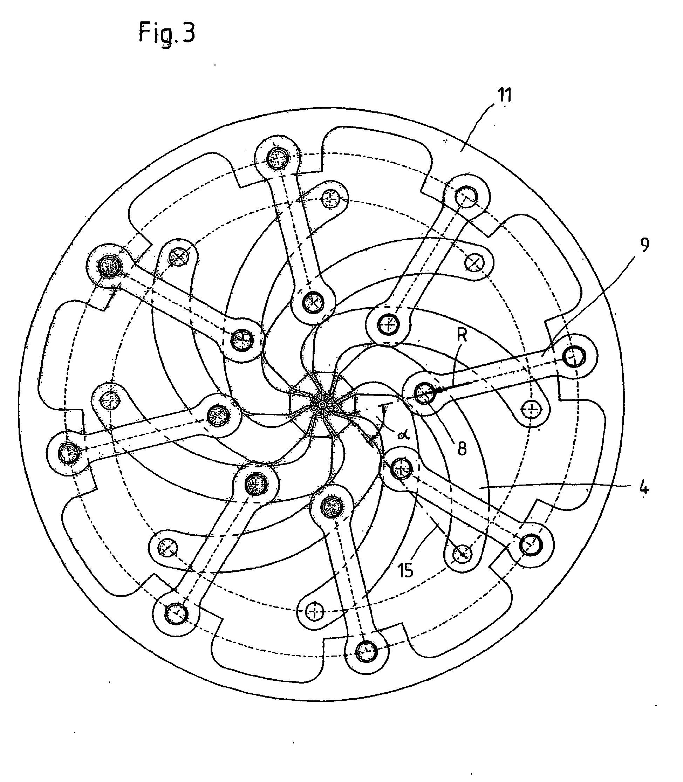

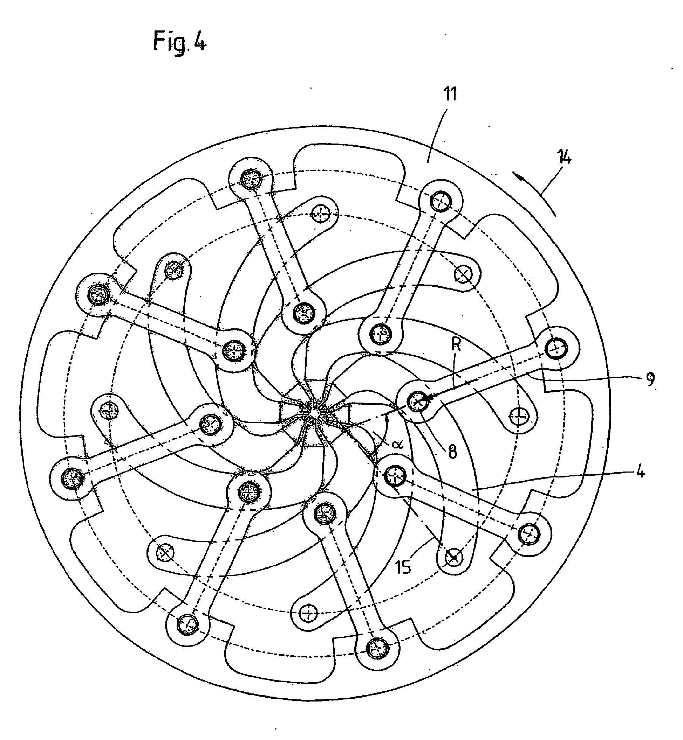

Figur 1 zeigt die Tamponpresse in geöffnetem Zustand und damit in jenem Zustand, in welchem der Tamponrohling 1 axial in den von den geöffneten Preßbacken gebildeten zylindrischen Raum eingeschoben wird. Figur 2 zeigt eine demgegenüber bereits mehr geschlossene Stellung der Preßbacken zur Ausübung einer Haltefunktion auf den Tamponrohling. Figur 3 zeigt die Tamponpresse im Zustand des maximalen Preßweges und damit auch des maximalen Preßdruckes innerhalb des Tampons. Die Preßbacken 2 sind weitestmöglich geschlossen. Da es nun in dem Zustand gemäß Figur 3 nahezu unmöglich ist, den vorgepreßten Vorformling wieder axial aus der Tamponpresse hinauszuschieben, erfolgt in einer letzten Stufe, die in Figur 4 dargestellt, ist ein wieder teilweises Öffnen bzw. Lüften der Preßbacken 2. In diesem Zustand kann der gebildete Vorformling mittels eines Stößels axial aus dem durch die Preßbacken gebildeten Formraum ausgeschoben werden.

Die in den Figuren 1 bis 4 schrittweise dargestellte Bewegung der Tamponpresse wird ausschließlich durch Verdrehen des ringförmigen Antriebselementes 11 in Richtung des in den Figuren 1 und 2 eingezeichneten Pfeiles 13 erzeugt. Der Pfeil 14 in Figur 4 symbolisiert die Öffnungsbewegung des ringförmiger Antriebselements 11 zum Zwecke des Lüftens der Tamponpresse. In dei gelüfteten Stellung gemäß Figur 4 läßt sich der Vorformling mittels des Stößels axial ausstoßen und in ein nachgeschaltetes Formwerkzeug einführen, in dem er seine Endform erhält. Dieses nachgeschaltete Formwerkzeug ist regelmäßig als sich konisch verjüngende Hülse ausgebildet, in der der Vorformling auf seinen endgültigen Durchmesser verformt wird.

In den Figuren 1 bis 4 ist jeweils der Winkel α eingezeichnet, welchen der als Übertragungselement dienende Druckstab 9 in bezug auf die gedachte Verbindungslinie 15 zwischen Lagerachse 5 und Preßbacke 2 einnimmt. In den Stellungen nach den Figuren 1 und 2 ist dieser Winkel α etwas größer als 90°, in den Stellungen nach den Figuren 3 und 4 etwas kleiner als 90°. Beträgt der Winkel α genau 90°, so ist in der hierbei eingenommenen Vorschubposition der Preßbacke 2 die Kraftübertragungsrichtung R des Druckstabes 9 gleichgerichtet zur Richtung der Bewegung der Preßbacke 2 zu diesem Zeitpunkt des Preßvorgangs. Bei allen anderen Winkellagen α fehlt diese Gleichrichtung. Ferner ist zu diesem Zeitpunkt und auch nur zu diesem einen Zeitpunkt des Preßvorgangs, der z. B. zwischen den Stellungen nach den Figuren 2 und 3 anzusiedeln ist, die Richtung R der von dem Druckstab 9 auf den Preßhebel 4 ausgeübten Krafteinleitung im wesentlichen quer, d. h. rechtwinklig zu der gedachten Verbindungslinie 15 zwischen Lagerachse 5 und Preßbacke 2. In mindestens einer Schwenklage des Preßhebels 4 erstreckt sich daher die Kraftübertragungsrichtung R des als Übertragungselementes dienenden Druckstabes 9 im wesentlichen quer zu dieser gedachten Verbindungslinie 15. In diesem Winkelbereich, der teilweise geringfügig kleiner und teilweise geringfügig größer als 90° ist, ist die Kraftübertragung optimal, d. h. es lassen sich bei moderater Antriebsleistung für das ringförmige Antriebselement 11 sehr hohe Preßkräfte auf den Preßhebel 4 und damit auf die dort rechtwinklig angeordnete Preßbacke 2 erzeugen. Im Zustand nach Fig. 3 ist die Richtung R nahezu direkt auf den Tampon 1 ausgerichtet.

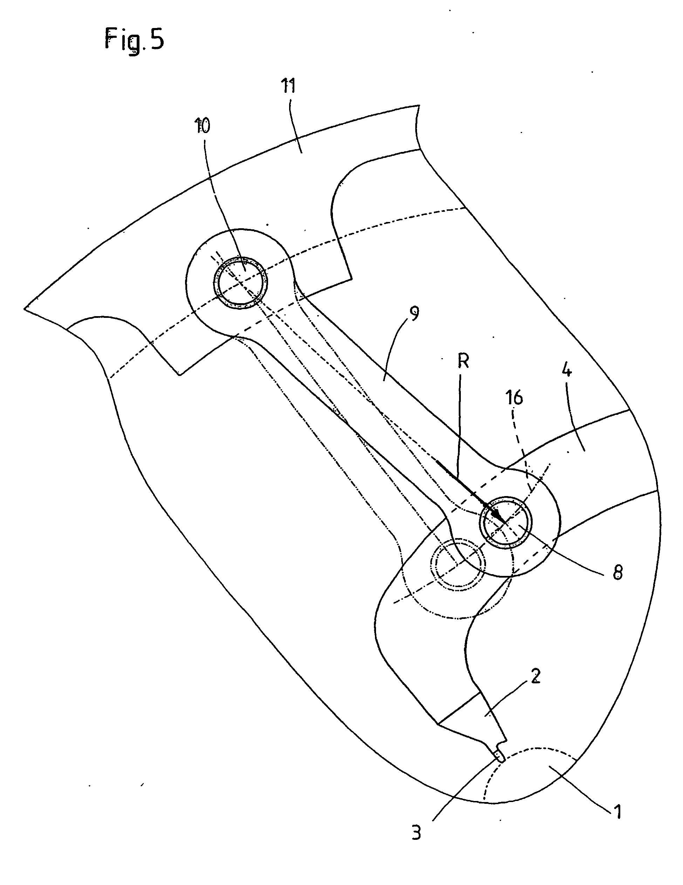

In Figur 5 ist dargestellt, daß durch Verlagerung des Drehzapfens 8 auf den Preßhebel 4 eine Einstellung der Tamponpresse auf den jeweiligen Anwendungsfall möglich ist, indem der Ort des Drehzapfens 8 in Längsrichtung des Preßhebels 4 verändert wird. Der hierbei optimale Ort kann bereits bei der Projektierung der Vorrichtung bestimmt werden, wodurch sich der konstruktive Aufwand zur Anpassung der Tamponpresse an das zu verarbeitende Material oder die Größe oder Festigkeit des Tampons gering halten läßt. Wird der Druckstab 9 z. B. mehr in Richtung auf die Lagerachse 5 an dem Preßhebel versetzt, ändern sich die Hebelverhältnisse, und es entsteht ein längerer Abschnitt des Preßhebels, an dem die Preßbacke 2 befestigt ist. Auf diese Weise erlangt der Preßhebel 4 in diesem Bereich eine größere Elastizität, und bei einem härter ausgerüsteten Baumwoll-/Zellwolle-Material für den Tampon oder bei höherem oder bei innerhalb der Produktion schwankendem Gewicht der Rohlinge kann der Preßhebel 4 geringfügig zurückfedern. Hierdurch entsteht dann ein etwas größerer Preßdurchmesser, wobei die Preßdichte des Tamponmaterials in etwa gleich bleibt.

Die genannte Einstellbarkeit des Ortes des Drehzapfens 8 kann auch konstruktiv verwirklicht werden, etwa durch ein Langloch in dem Preßhebel 4, dessen Mittellinie 16 in Figur 5 eingezeichnet ist. Durch Versetzen entlang dieser Mittellinie 16 läßt sich die gewünschte Preßgeometrie auch nachträglich mit wenig Aufwand verändern.

### Bezugszeichenliste:

- 1: Tampon
- 2: Preßbacken
- 3: Preßschneide
- 4: Preßhebel
- 5: Lagerachse, Gelenk
- 6: Stüztring
- 7: Kreis
- 8: Drehzapfen
- 9: Druckstab
- 10: Drehzapfen
- 11: Antriebselement
- 12: Drehachse
- 13: Pfeil
- 14: Pfeil
- 15: Verbindungslinie
- 16: Mittellinie
- α: Winkel
- R: Richtung der Krafteinleitung

## Patentansprüche

1. Verfahren zum Herstellen eines Tampons, bei dem in einer Tamponpresse mit radial beweglichen Pressbacken streifenförmige Abschnitte eines zylindrischen Rohlings aus saugfähigem Material bis zur Bildung eines Vorformlings gepresst werden, der sich, im Querschnitt gesehen, aus einem zentralen Tamponkern und sich von dem Tamponkern radial nach aussen erstreckenden Längsrippen zusammensetzt, die durch Längsrillen voneinander getrennt sind, **dadurch gekennzeichnet, dass** die Kraft zur Bewegung jeder Pressbacke (2) über ein zu der Pressbacke (2) gelenkiges Druckübertragungselement (9) auf die jeweilige Pressbacke (2) übertragen wird, und dass die Richtung (R) der Krafteinleitung zu einem Zeitpunkt des Pressvorgangs gleichgerichtet zu der Bewegung der Pressbacke (2) ist.

2. Verfahren nach Anspruch 2, **dadurch gekennzeichnet, dass** die streifenförmigen Abschnitte durch Verschwenken von mit den Pressbacken (2) versehenen, gelenkig mit dem Druckübertragungselement (9) verbundenen Presshebeln (4) um Gelenke (5) gepresst werden, wobei die Lage aller Gelenke (5) unveränderlich zueinander ist, und dass zu dem genannten Zeitpunkt des Pressvorgangs die Richtung (R) der Krafteinleitung quer zu der gedachten Verbindungslinie (15) zwischen Gelenk (5) und Pressbacke (2) ist.

3. Verfahren nach Anspruch 2, **dadurch gekennzeichnet, dass** die Kraft zum Verschwenken des Presshebels (4) an einem Ort des Presshebels zwischen seinem Gelenk (5) und seiner Pressbacke (2) in den Presshebel eingeleitet wird.

4. Verfahren nach einem der vorangehenden Ansprüche, **dadurch gekennzeichnet, dass** bei maximalem Vorschub der Pressbacken die Krafteinleitung (Richtung R) radial auf den Vorformling (1) ausgerichtet ist.

5. Verfahren nach einem der Ansprüche 2 bis 4, **dadurch gekennzeichnet, dass** die Lagerachsen (5) für die gelenkige Lagerung der Presshebel (4) feststehend sind.

6. Verfahren nach einem der Ansprüche 1 bis 5, **dadurch gekennzeichnet, dass** die Betätigung aller Pressbacken (2) durch Verdrehen eines gemeinsamen Antriebselements (11) um eine Drehachse (12) erfolgt, die mit der Mittelachse des Vorformlings (1) zusammenfällt.

7. Vorrichtung zum Herstellen eines Tampons, mit einer Tamponpresse aus über den Umfang eines zu pressenden Tamponrohlings verteilt angeordneten, vor- und zurückbeweglichen und mit hervorstehenden Pressschneiden versehenen Pressbacken (2), **dadurch gekennzeichnet, dass** die Pressbacken (2) in gelenkiger Verbindung mit dem einen Ende eines Druckübertragungselementes (9) stehen, dessen anderes Ende mit einem Antrieb in Verbindung steht, und dass in einer Vorschubposition der jeweiligen Pressbacke (2) die Kraftübertragungsrichtung (R) des Druckübertragungselementes (9) gleichgerichtet zu der Bewegung der Pressbacke (2) ist.

8. Vorrichtung nach Anspruch 7, **dadurch gekennzeichnet, dass** die Pressbacken (2) jeweils am Ende eines um eine Lagerachse (5) schwenkbaren Presshebels (4) angeordnet sind, wobei die Lage aller Lagerachsen (5) der Presshebel unveränderbar zueinander ist, und dass sich in einer Schwenklage des Presshebels (4) die Kraftübertragungsrichtung (R) des Druckübertragungselementes (9) quer zu der gedachten Verbindungslinie (15) zwischen Lagerachse (5) und Pressbacke (2) erstreckt.

9. Vorrichtung nach Anspruch 8, **dadurch gekennzeichnet, dass** die Presshebel (4) zwischen ihrer Lagerachse (5) und ihrer Pressbacke (2) gelenkig mit dem Druckübertragungselement (9) verbunden sind.

10. Vorrichtung nach einem der Ansprüche 7 bis 9, **dadurch gekennzeichnet, dass** die Kraftübertragungsrichtung (R) des Druckübertragungselements (9) bei maximalem Vorschub des Presshebels (4) radial auf den Tampon (1) ausgerichtet ist.

11. Vorrichtung nach einem der Ansprüche 7 bis 10, **dadurch gekennzeichnet, dass** das Druckübertragungselement ein Druckstab (9) ist, dessen anderes Ende, ebenso wie die anderen Enden der übrigen Druckstäbe (9) der Tamponpresse, gelenkig mit einem gemeinsamen Antriebselement (11) verbunden ist.

12. Vorrichtung nach Anspruch 11, **dadurch gekennzeichnet, dass** das gemeinsame Antriebselement (11) ein drehbarer Ring ist, dessen Drehachse (12) mit der Mittelachse des Tampons (1) zusammenfällt.

13. Vorrichtung nach Anspruch 12, **dadurch gekennzeichnet, dass** die Presshebel (4) sowie die mit diesen gelenkig verbundenen Druckstäbe (9) innerhalb des Antriebselements (11) angeordnet sind.

14. Vorrichtung nach einem der Ansprüche 8 bis 13, **gekennzeichnet durch** einen feststehenden Stützring (6), an dem die Lagerachsen (5) der Presshebel (4) angeordnet sind.

15. Vorrichtung nach einem der Ansprüche 9 bis 14, **dadurch gekennzeichnet, dass** das den Presshebel (4) mit dem Druckübertragungselement (9) verbindende Gelenk (8) näher an der Pressbacke (2), als an der Lagerachse (5) sitzt.

16. Vorrichtung nach einem der Ansprüche 8 bis 15, **dadurch gekennzeichnet, dass** der Ort des den Presshebel (4) mit dem Druckübertragungselement (9) verbindenden Gelenks (8) in Längsrichtung des Presshebels (4) einstellbar ist.

## Claims

1. Method of producing a tampon, whereby strip-shaped portions of a cylindrical blank made from absorbent material are pressed in a tampon press with radially displaceable pressing jaws to form a preform made up of a central tampon core and longitudinal ribs extending radially outwards from the tampon core as viewed in cross-section, which are separated from one another by longitudinal grooves, **characterised in that** the force for moving each pressing jaw (2) is transmitted to the respective pressing jaw (2) via a pressure transmitting means (9) articulatingly linked to the pressing jaw (2), and the direction (R) in which the force is transmitted is aligned with the movement of the pressing jaw (2) at an instant of the pressing operation.

2. Method as claimed in claim 2, **characterised in that** the strip-shaped portions are pressed by pivoting pressing levers (4) fitted with the pressing jaws (2) linked to the pressure transmitting element (9) so as to articulate about articulated joints (5), and the position of all the articulatedjoints (5) relative to one another does not change, and at said instant of the pressing operation, the force is transmitted in the direction (R) extending transversely to the imaginary connecting line (15) between the articulated joint (5) and pressing jaw (2).

3. Method as claimed in claim 2, **characterised in that** the force for pivoting the pressing lever (4) is transmitted to a point of the pressing lever between its articulated joint (5) and its pressing jaw (2).

4. Method as claimed in one of the preceding claims, **characterised in that** the transmitted force (direction R) is directed onto the preform (1) radially when the pressing jaws are in the maximum forward position.

5. Method as claimed in one of claims 2 to 4, **characterised in that** the bearing pins (5) providing an articulated mounting for the pressing levers (4) are stationary.

6. Method as claimed in one of claims 1 to 5, **characterised in that** all the pressing jaws (2) are operated by rotating a common drive element (11) about an axis of rotation (12) which coincides with the mid-axis of the preform (1).

7. Device for producing a tampon with a tampon press comprising pressing jaws (2) distributed around the circumference of a tampon blank to be pressed having protruding pressing edges which can be moved backwards and forwards, **characterised in that** the pressing jaws (2) are articulatingly connected to one end of a pressure transmitting element (9), the other end of which is connected to a drive, and when the respective pressing jaw (2) is in a forward position, the direction (R) of the force transmitted by the pressure transmitting element (9) is aligned with the movement of the pressing jaw (2).

8. Device as claimed in claim 7, **characterised in that** the pressing jaws (2) are respectively disposed at the end of a pressing lever (4) which is able to pivot about a bearing pin (5), and the position of all the bearing pins (5) of the pressing levers relative to one another does not change, and the direction (R) in which the force of the pressure transmitting element (9) is transmitted when the pressing lever (4) is in a pivoted position extends transversely to the imaginary connecting line (15) between the bearing pin (5) and pressing jaw (2).

9. Device as claimed in claim 8, **characterised in that** the pressing levers (4) are articulatingly connected to the pressure transmitting element (9) between their bearing pin (5) and their pressing jaw (2).

10. Device as claimed in one of claims 7 to 9, **characterised in that** the direction (R) in which the force of the pressure transmitting element (9) is transmitted is directed radially onto the tampon (1) when the pressing lever (4) is in the maximum forward position.

11. Device as claimed in one of claims 7 to 10, **characterised in that** the pressure transmitting element is a pressing bar (9), the other end of which, as well as the other ends of the other pressing bars (9) of the tampon press, are articulatingly linked to a common drive element (11).

12. Device as claimed in claim 11, **characterised in that** the common drive element (11) is a rotatable ring, the axis of rotation (12) of which coincides with the mid-axis of the tampon (1).

13. Device as claimed in claim 12, **characterised in that** the pressing levers (4) and the pressing bars (9) articulatingly linked to it are disposed inside the drive element (11).

14. Device as claimed in one of claims 8 to 13, **characterised by** a stationary support ring (6), on which the bearing pins (5) of the pressing levers (4) are disposed.

15. Device as claimed in one of claims 9 to 14, **characterised in that** the articulated joint (8) connecting the pressing lever (4) to the pressure transmitting element (9) sits closer to the pressing jaw (2) than to the bearing pin (5).

16. Device as claimed in one of claims 8 to 15, **characterised in that** the point at which the articulated joint (8) connects the pressing lever (4) to the pressure transmitting element (9) can be adjusted in the longitudinal direction of the pressing lever (4).

## Revendications

1. Procédé destiné à la fabrication d'un tampon, dans lequel, dans une presse pour tampons avec des mâchoires de pressage radialement mobiles, des tronçons en forme de bande d'une ébauche cylindrique en matériau absorbant sont comprimés jusqu'à former une préforme qui, vue en coupe transversale, est formée par une âme centrale du tampon et des nervures longitudinales qui s'étendent radialement vers l'extérieur à partir de l'âme du tampon et qui sont séparées les unes des autres par des cannelures longitudinales, **caractérisé en ce que** la force pour déplacer chaque mâchoire de pressage (2) est transmise sur la mâchoire de pressage (2) respective par un élément de transmission de la pression (9), articulé par rapport à la mâchoire de pressage (2), et **en ce que** la direction (R) d'introduction de la force, à un instant du processus de pressage, est orientée dans le même sens que le mouvement de la mâchoire de pressage (2).

2. Procédé selon la revendication 2, **caractérisé en ce que** les tronçons en forme de bande sont comprimés sous l'effet du pivotement de leviers de pressage (4) autour d'articulations (5), lesquels sont munis des mâchoires de pressage (2) et assemblés de manière articulée à l'élément de transmission de la pression (9), toutes les articulations (5) étant positionnées de manière invariable les unes par rapport aux autres, et **en ce que**, la direction (R) d'introduction de la force, à l'instant donné du processus de pressage, est orientée transversalement à la ligne de jonction (15) imaginaire entre l'articulation (5) et la mâchoire de pressage (2).

3. Procédé selon la revendication 2, **caractérisé en ce que** la force pour faire pivoter le levier de pressage (4) est introduite dans le levier de pressage à un emplacement du levier de pressage entre son articulation (5) et sa mâchoire de pressage (2).

4. Procédé selon l'une quelconque des revendications précédentes, **caractérisé en ce que**, dans le cas d'un mouvement d'avance maximal des mâchoires de pressage, l'introduction de la force (direction R) est orientée radialement vers la préforme (1).

5. Procédé selon l'une quelconque des revendications 2 à 4, **caractérisé en ce que** les axes de montage (5) pour le montage articulé des leviers de pressage (4) sont fixes.

6. Procédé selon l'une quelconque des revendications 1 à 5, **caractérisé en ce que** l'actionnement de toutes les mâchoires de pressage (2) est assuré par la rotation d'un élément d'entraînement (11) commun autour d'un axe de rotation (12), qui coïncide avec l'axe médian de la préforme (1).

7. Dispositif destiné à la fabrication d'un tampon, comportant une presse pour tampons formée par des mâchoires de pressage (2), réparties sur le pourtour d'une ébauche de tampon à comprimer, mobiles vers l'avant et l'arrière et munies d'arêtes de coupe saillantes, **caractérisé en ce que** les mâchoires de pressage (2) sont assemblées de manière articulée avec l'une des extrémités d'un élément de transmission de la pression (9), dont l'autre extrémité est assemblée à un système d'entraînement, et **en ce que**, dans une position d'avance de chacune des mâchoires de pressage (2), la direction de transmission de la pression (R) de l'élément de transmission de la pression (9) est orientée dans le même sens que le mouvement de la mâchoire de pressage (2).

8. Dispositif selon la revendication 7, **caractérisé en ce que** les mâchoires de pressage (2) sont agencées chacune à l'extrémité d'un levier de pressage (4) apte à pivoter autour d'un axe de montage (5), tous les axes de montage (5) des leviers de pressage étant positionnés de manière invariable les uns par rapport aux autres, et **en ce que**, dans une position de pivotement du levier de pressage (4), la direction de transmission de la pression (R) de l'élément de transmission de la pression (9) est orientée transversalement à la ligne de jonction (15) imaginaire entre l'axe de montage (5) et la mâchoire de pressage (2).

9. Dispositif selon la revendication 8, **caractérisé en ce que** les leviers de pressage (4), entre leur axe de montage (5) et leur mâchoire de pressage (2), sont assemblés de manière articulée avec l'élément de transmission de la pression (9).

10. Dispositif selon l'une quelconque des revendications 7 à 9, **caractérisé en ce que**, dans le cas d'un mouvement d'avance maximal du levier de pressage (4), la direction de transmission de la pression (R) de l'élément de transmission de la pression (9) est orientée radialement vers le tampon (1).

11. Dispositif selon l'une quelconque des revendications 7 à 10, **caractérisé en ce que** l'élément de transmission de la pression est une tige de pression (9), dont l'autre extrémité, de même que les autres extrémités des autres tiges de pression (9) de la presse pour tampons, est assemblée de manière articulée à un élément d'entraînement (11) commun.

12. Dispositif selon la revendication 11, **caractérisé en ce que** l'élément d'entraînement (11) commun est un anneau rotatif, dont l'axe de rotation (12) coïncide avec l'axe médian du tampon (1).

13. Dispositif selon la revendication 12, **caractérisé en ce que** les leviers de pressage (4), ainsi que les tiges de pression (9), assemblées de manière articulée à ces derniers, sont disposés à l'intérieur de l'élément d'entraînement (11).

14. Dispositif selon l'une quelconque des revendications 8 à 13, **caractérisé par** un anneau de support (6) fixe, sur lequel sont disposés les axes de montage (5) des leviers de pressage (4).

15. Dispositif selon l'une quelconque des revendications 9 à 14, **caractérisé en ce que** l'articulation (8), qui relie le levier de pressage (4) avec l'élément de transmission de la pression (9), est disposée plus près de la mâchoire de pressage (2) que de l'axe de montage (5).

16. Dispositif selon l'une quelconque des revendications 8 à 15, **caractérisé en ce que** l'emplacement de l'articulation (8), qui relie le levier de pressage (4) avec l'élément de transmission de la pression (9), est réglable dans la direction longitudinale du levier de pressage (4).
